Europäisches Patentamt

European Patent Office

Office européen des brevets

(11) Veröffentlichungsnummer: **0 234 503**
**B1**

(12) **EUROPÄISCHE PATENTSCHRIFT**

(45) Veröffentlichungstag der Patentschrift: **12.09.90**

(21) Anmeldenummer: **87102342.0**

(22) Anmeldetag: **19.02.87**

(51) Int. Cl.⁵: **C 07 C 49/16, C 07 C 45/63**

(54) **Verfahren zur Herstellung von 1,1,3-Trichloraceton.**

(30) Priorität: **20.02.86 DE 3605484**

(43) Veröffentlichungstag der Anmeldung:
**02.09.87 Patentblatt 87/36**

(45) Bekanntmachung des Hinweises auf die
Patenterteilung:
**12.09.90 Patentblatt 90/37**

(84) Benannte Vertragsstaaten:
**AT CH DE FR GB IT LI**

(56) Entgegenhaltungen:
**US-A-4 251 467**

(73) Patentinhaber: **WACKER-CHEMIE GMBH**
**Prinzregentenstrasse 22**
**D-8000 München 22 (DE)**

(72) Erfinder: **Deinhammer, Wolfgang, Dr.Dipl.-Ing.**
**Röntgenstrasse 32**
**D-8263 Burghausen (DE)**
Erfinder: **Petersen, Hermann, Dr. Dipl.-Chem.**
**Karl-Gross-Strasse 11**
**D-8263 Burghausen (DE)**

**Die Akte enthält technische Angaben, die nach dem Eingang der Anmeldung eingereicht wurden und die nicht in dieser Patentschrift enthalten sind.**

Courier Press, Leamington Spa, England.

**Beschreibung**

Die Herstellung von chlorierten Produkten des Acetons ist seit langem bekannt, wobei in Abhängigkeit von Art und Menge des Chlorierungsmittels und der Temperatur mono-, di-, tri- und höherchlorierte Produkte gebildet werden.

Unter diesen bekannten Produkten kommt dem 1,1,3-Trichloraceton besondere Bedeutung zu, das als Zwischenprodukt beispielsweise für die Synthese von Folsäure Verwendung findet.

Die einfachste Herstellungsmethode ist die direkte Einwirkung von Chlor auf Aceton. Hierbei ist jedoch mit der Bildung unerwünschter Nebenprodukte zu rechnen, da der bei der Chlorierungsreaktion frei werdende Chlorwasserstoff Kondensationsreaktionen von noch nicht umgesetzten Aceton begünstigt. Nach dem aus der US—A—2 635 118 bekannten Verfahren zur Herstellung von Di- und Trichloracetonen wird daher die Chlorierung von Aceton unter Ausschluß von Wasser bei Temperaturen bis zu 100°C unter Verwendung des flüssigen Reaktionsgemisches selbst, das aus Di- und Trichloracetonen besteht, als Lösungsmittel durchgeführt. Außerdem wird zweckmäßig mit einem geringen Chlorüberschuß gearbeitet. Bei diesem Verfahren können intermolekulare Kondensationen des Acetons weitgehend unterdrückt und hohe Ausbeuten an chloriertem Produkt, bezogen auf eingesetztes Aceton, erreicht werden. Die Endprodukte sind jedoch Rohgemische aus Di- und Trichloraceton, wobei der Anteil der Trichloracetone, die selbst Rohgemische aus 1,1,1- und 1,1,3-Trichloraceton sind, bei Einsatz entsprechend höherer Chlormengen zunimmt. Die selektive Herstellung von 1,1,3-Trichloraceton ist nach diesem Verfahren nicht möglich.

Nach dem aus der US—A—3 346 646 bekannten Verfahren, das sich mit der selektiven Halognierung von Ketonen befaßt, führt die Einwirkung von Chlor auf 1-Chlor- oder 1,1-Dichloraceton oder Gemischen hiervon in wäßrig-alkalischem Reaktionsmedium vorwiegend zur Bildung von 1,1,1-Trichloraceton.

Die Chlorierung von Aceton in Gegenwart von sauren und basischen Katalysatoren mit dem Ziel 1,1,3-Trichloaceton möglichst frei von Isomeren durch direkte Chlorierung von Aceton zu erhalten wurde von L. V. Bugrova et al. in Zh. Prikl. Khim. 1973, vol. 46(7), S. 1529—1533, ref. in C.A. vol. 79 (1973): 91499 q untersucht. Dabei wurde Aceton unter verschiedenen Bedingungen mit und ohne Katalysatoren chloriert. Die besten Ergebnisse wurden bei extrem langsamem Einleiten von Chlor in genau äquimolaren Mengen in Gegenwart von Pyridin oder substituierten Aminen, vorzugsweise Diethylamin als Katalysatoren bei Temperaturen von 25° bis 30°C erzielt, wobei das Chlorierungsgemisch 50,6 bis 57,2% 1,1,3-Trichloraceton enthält.

Aus einem derartigen Chlorierungsgemisch kann das 1,1,3-Trichloraceton üblicherweise durch Rektifikation gewonnen werden. Da jedoch die Nebenprodukte 1,3-Dichlor-, 1,1,3,3- und 1,1,1,3-Tetrachloraceton aufgrund ihrer ähnlichen Siedepunkte praktisch technisch nicht abtrennbar sind, wird das 1,1,3-Isomere nur mit einer Reinheit von etwa 80 bis 90% erhalten. Eine verbesserte Reinigungswirkung kann durch Extraktion des Hydrats aus wäßriger Lösung erreicht werden, die aber sehr aufwendig und verlustreich ist (vgl. SU-251 565, ref. in C.A. vol. 87 (1977): 22410 g).

"In der US—A—4,251,407 wird ein Verfahren zur Herstellung von symmetrischem 1,3-Chloraceton beschrieben, bei dem molekulares Chlor mit einer wäßrigen Mischung aus Aceton und/ oder Monochloraceton und einem Jod enthaltenden Promotor in Berührung gebracht wird. Für die Selektivität dieses Verfahrens in Richtung auf die Bildung der symmetrischen 1,3-Verbindung ist dabei das Vorhandensein eines wasserlöslichen Jod enthaltenden Promotors in Verbindung mit Wasser als Reaktionsmedium von entscheidender Bedeutung. Unter diesen Bedingungen soll durch sterische Hinderung die Bildung von 1-Jod-1-chloraceton verhindert werden, während im Gegensatz dazu durch direkte Chlorierung die Bildung von 1,1-Dichloraceton begünstigt wird. Darüberhinaus spielt die Temperatur ein wichtige Rolle, denn mit steigender Temperatur nimmt die Selektivität des 1,3-Dichloracetons ab, well die Reaktionsfähigkeit des molekularen Chlors zunimmt und dieses mit Monochloraceton direkt in statistisch verteilter Weise ohne die Bildung des jodhaltigen Zwischenprodukts reagiert, wobei bei höheren Temperaturen auch etwas Trichloraceton gebildet wird. Diese Patentschrift vermittelt demnach die Lehre, daß durch Auswahl eines wasserlöslichen Jod enthaltenden Promotors in Kombination mit einem wäßrige Reaktionsmedium bei der Chlorierung von Aceton und/oder Monochloraceton die Selektivität des an sich schwer zugänglichen 1,3-Dichloraceton gesteigert werden kann. für die Steigerung der Selektivität von 1,1,3-Trichloraceton kann hieraus jedoch keine Anregung entnommen werden, denn in diesem Fall ist 1,3-Dichloraceton ein unerwünschtes Nebenprodukt, das ebenso wie die beiden Tetrachloracetone destillativ nicht abtrennbar ist und deren Bildung daher möglichst unterdrückt werden soll".

Es stellt sich somit die Aufgabe, das Verfahren der Chlorierung von Aceton oder dessen Mono- oder Dichlorderivaten unter Ausschluß von Wasser bei Temperaturen bis zu 100°C so zur verbessern, daß die Herstellung von 1,1,3-Trichloaceton mit hoher Selektivität und guter Ausbeute ermöglicht und die Bildung von Chlorierungsprodukten mit ähnlichen Siedepunkten wie das 1,1,3-Trichloaceton praktisch weitgehend unterdrückt wird.

Diese Aufgabe wird erfindungsgemäß dadurch gelöst, daß Aceton, Chloraceton oder 1,1-Dichloraceton, einzeln oder im Gemisch bei Temperaturen im Bereich von 10° bis 80°C mit höchstens äquimolaren Chlormengen in Gegenwart von Jod oder einer im Reaktionsgemisch löslichen Jodverbindung umgesetzt werden.

Das erfindungsgemäße Verfahren kann sowohl diskontinuierlich als auch kontinuierlich durchgeführt werden. Als Reaktoren können beispielsweise Rührwerke, Reaktionskreisläufe oder Blasensäulen verwendet werden.

Als Ausgangsprodukte werden definitionsgemäß Aceton, Monochloraceton oder 1,1-Dichloraceton einzeln oder im Gemisch eingesetzt. Vorteilhaft wird das Verfahren bei Temperaturen im Bereich von 15° bis 50°C durchgeführt. Die Chloreinleitungsgeschwindigkeit hat praktisch keinen Einfluß auf die Seletivität. Sie kann in Abhängigkeit von der Reaktorbauart 0,01 bis 2 Mol Chlor pro Mol Ausgangsprodukt und Stunde betragen.

Die Menge des einzuleitenden Chlors ist selbstverständlich von der Art des eingesetzten Ausgangsmaterial abhängig. Die Ausdrucksweise "höchstens äquimolare Chlormengen" ist daher so zu versehen, daß je Mol Aceton 2,5 bis 3 Mol Chlor, je Mol Monochloraceton 1,5 bis 2 Mol Chlor und je Mol 1,1-Dichloraceton 0,2 bis 1 Mol Chlor erforderlich sind. Vorzugsweise wird mit einem geringen Chlorunterschuß gearbeitet, worunter zu verstehen ist, daß je Mol Aceton 2,6 bis 2,9 Mol Chlor, je Mol Monochloraceton 1,6 bis 1,9 Mol Chlor und je Mol 1,1-Dichloraceton 0,3 bis 0,8 Mol Chlor eingeleitet werden.

Als Katalysatoren, die die Selektivität in Richtung der gewünschten 1,1,3-Trichlorverbindung ermöglichen, werden definitionsgemäß Jod oder im Reaktionsgemisch lösliche Jodverbindungen verwendet. Die eingesetzte Menge wird hierbei voteilhaft so bemessen, daß der Gehalt an Jod im Chlorierungsprodukt 0,5 bis 10 Gewichtsprozent beträgt.

Das eingesetzt Jod liegt nach der Chlorierung vorweigend als 1,1-Dichlor-3-jodaceton vor. Diese Verbindung kann bei der anschließenden Reinigungsdestillation als Hochsieder leicht vom 1,1,3-Trichloraceton abgetrennt und als Katalysator für weitere Ansätze wieder verwendet werden. Der Jodverbrauch ist demnach äußerste gering und beschränkt sich nur auf den Ersatz von Verlusten bei der Rezirkulierung. Als jedhaltige Katalysatoren können außer dem genannten 1,1-Dichlor-3-jodaceton auch Jodaceton, 1-Jod-3-chloraceton sowie Jodchlorid, Jodtrichlorid oder Jodwasserstoff verwendet werden. Entscheidend ist hierbei nur, daß die Jodverbindung in dem vorhandenen Reaktionsgemisch gut löslich ist, damit für eine ausreichende Verteilung gesorgt werden kann.

Bei der anschließenden Reinigungsdestillation kann das 1,1-Dichloraceton, das als Nebenprodukt gebildet oder als Ausgangsmaterial nicht vollständig umgesetzt worden ist, aufgrund seines Siedepunktunterschiedes von 60°C zum 1,1,3-Trichloraceton leicht destillativ abgetrennt und bei einem nächsten Chlorierungsansatz wieder zugefügt werden, unabhängig davon, welches Ausgangsprodukt hierbei verwendet wird. Ein weiteres Nebenprodukt ist 1,1,1-Trichloraceton, des ebenfalls leicht destillativ abgetrennt werden kann (Siedepunktsunterschied von 45°C zum 1,1,3-Trichloraceton).

Bei Durchführung des erfindungsgemäßen Verfahrens mit 1,1-Dichloraceton als Ausgangsmaterial wird nach der Reinigung durch franktionierte Destillation 1,1,3-Trichloraceton mit einer Reinheit von 97 bis 98 Gewichtsprozent erhalten in einer Gesamtausbeute von etwa 85% der Theorie, das heißt einschließlich der Destillationsverluste. Bei Einsatz von Aceton oder Chloraceton sinkt zwar die Reinheit bis auf 96 Gewichtsprozent ab, durch die verringerte Bildung von 1,1,1-Trichloraceton ist es jedoch möglich, eine Gesamtausbeute von mehr als 65% der Theorie zu erzielen.

In den folgenden Beispielen wird das erfindungsgemäße Verfahren näher erläutert.

Beispiel 1

In einem mit Rührer, Gaseinleitungsrohr und Rückflußkühler ausgerüstetem Rundkolben von 250 ml Fassungsvermögen wurden in ein Gemisch aus 190,5 g 1,1-Dichloroaceton und 6 g Jod bei 30°C 63 g Chlor innerhalb von 3,5 Stunden eingeleitet. Die Analyse des Reaktionsgemisches ergab:

    34   Gew.-% 1,1-Dichloraceton
    6,5  Gew.-% 1,1,1-Trichloraceton
    53   Gew.-% 1,1,3-Trichloraceton
    0,7  Gew.-% Tetrachloracetone und
    4    Gew.-% 1,1-Dichlor-3-jodaceton.

Dieses Gemisch wurde über eine 30 cm hohe, mit 4 mm Raschigringen gefüllte Kolonne mit einem Rücklaufverhältnis von 1:10 bei einem Druck von 2660 Pa fraktioniert. Als Hauptfraktion wurde 1,1,3-Trichloraceton mit einer Reinheit von 98 Gewichtsprozent erhalten.

Beispiel 2

Unter den gleichen Bedingungen wie in Beispiel 1 beschrieben, wurden in ein Gemisch aus 112 g 1,1-Dichloraceton, 0,5 g Jod, 78 g des Vorlaufs und 20 g des Destillationsrückstandes, die bei der Destillation gemäß Beispiel 1 erhalten wurden, 63 g Chlor eingeleitet. Bei der fraktionierten Destillation wurden 125 g 1,1,3-Trichloraceton mit einer Reinheit von 97 Gewichtsprozent erhalten, das entspricht 85% der Theorie, bezogen auf das in Beispiel 2 eingesetzte Dichloraceton.

Beispiel 3

Unter den gleichen Bedingungen wie in Beispiel 1 beschrieben, wurden in ein Gemisch aus 190,5 g 1,1-Dichloraceton und 6,5 g Jod 103 g Chlor innerhalb von 7 Stunden eingeleitet. Das Rohprodukt enthielt 71 Gewichtsprozent 1,1,3-Trichloraceton.

Beispiel 4

Unter den gleichen Bedingungen wie in Beispiel 1 beschrieben, wurden in en Gemisch aus 91,5 g Chloraceton und 6 g Jod 135 g Chlor innerhalb von 6 Stunden eingeleitet.
Die Analyse des Reaktionsgemisches ergab:

25 Gew.-% 1,1-Dichloraceton
5,5 Gew.-% 1,1,1-Trichloraceton
0,8 Gew.-% 1,3-Dichloraceton
62 Gew.-% 1,1,3-Trichloraceton
2,1 Gew.-% Tetrachloracetone und
4 Gew.-% 1,1-Dichlor-3-jodaceton.

Beispiel 5

Unter den gleichen Bedingungen wie in Beispiel 1 beschrieben, wurden in ein Gemisch aus 14,5 g Aceton und 1,4 g Jod bei 20° bis 30°C 20 g Chlor innerhalb von 10 Minuten und weitere 36 g Chlor innerhalb von 4 Stunden eingeleitet. Das Rohprodukt enthielt 76 Gewichtsprozent 1,1,3-Trichloaceton.

Vergleichsbeispiel

Unter den gleichen Bedingungen wie in Beispiel 1 der US—A—2,635,118 beschrieben, wurden in ein mit Glaskörpern gefülltes Reaktionsrohr 149 g Aceton und 547 g Chlor pro Stunde eingeleitet. Nach 5,25 Stunden wurde eine Probe des Reaktionsgemisches analysiert:

1,5 Gew.-% Chloraceton
23 Gew.-% 1,1-Dichloraceton
11 Gew.-% 1,3-Dichloraceton
2 Gew.-% 1,1,1-Trichloraceton
43 Gew.-% 1,1,3-Trichloraceton
13 Gew.-% 1,1,3,3-Tetrachloraceton
5 Gew.-% 1,3,3,3-Tetrachloraceton und
0,4 Gew.-% Pentachloraceton.

Die Ausbeute an 1,1,3-Trichloraceton berechnet als 100%-iges Produkt, betrug 37% (bezogen auf eingesetztes Aceton). Dieses Produkt wurde über eine 30 cm hohe, mit 4 mm Raschigringen gefüllte Kolonne mit einem Rücklaufverhältnis von 1:10 fraktioniert.

Als Hauptfraktion wurde ein Gemisch mit folgender Zusammensetzung erhalten:

0,4 Gew.-% 1,1,1-Trichloaceton
13 Gew.-% 1,3-Dichloraceton
58 Gew.-% 1,1,3-Trichloraceton
20 Gew.-% 1,1,3,3-Tetrachloraceton und
8 Gew.-% 1,3,3,3-Tetrachloraceton.

Dieses Beispiel beweist, daß ohne die Mitverwendung des erfindungsgemäßen Katalysators und bei der relativ hohen Reaktionstemperatur der Anteil von destillativ nicht abtrennbaren Chlorierungsprodukten so hoch ist, daß eine selektive Herstellung von 1,1,3-Trichloraceton nicht möglicht war.

**Patentansprüche**

1. Verfahren zur Herstellung von 1,1,3-Trichloraceton durch Chlorierung von Aceton oder dessen Mono- oder Dichlorderivativen unter Ausschluß von Wasser bei Temperaturen bis zu 100°C, dadurch gekennzeichnet, daß Aceton, Chloraceton oder 1,1-Dichloraceton, einzeln oder im Gemisch bei Temperaturen im Bereich von 10° bis 80°C mit höchsten äquimolaren Chlormengen in Gegenwart von Jod oder einer im Reaktionsgemisch löslichen Jod enthaltenden Verbindung umgesetzt werden.

2. Verfahren nach Anspruch 1, dadurch gekennzeichnet, daß die Chlorierung bei Temperaturen im Bereich von 15° bis 50°C durchgeführt wird.

3. Verfahren nach Anspruch 1, dadurch gekennzeichnet, daß die Chlorierung mit einem geringen Chlorunterschuß durchgeführt wird.

4. Verfahren nach Anspruch 3, dadurch gekennzeichnet, daß je Mol Aceton 2,6 bis 2,9 Mol Chlor, je Mol eingesetztem Monochloraceton 1,6 bis 1,9 Mol Chlor und je Mol eingesetztem 1,1-Dichloraceton 0,3 bis 0,8 Mol Chlor verwendet werden.

5. Verfahren nach Anspruch 1, dadurch gekennzeichnet, daß als Jod enthaltende Verbindungen Jodaceton, 1-Jod-3-chloraceton, 1,1-Dichlor-3-jodaceton, Jodchlorid, Jodtrichlorid oder Jodwasserstoff verwendet werden.

**Revendications**

1. Procédé pour préparer la trichloro-1,1,3 acétone par chloration de l'acétone ou de ses dérivés monochlorés ou dichlorés, à l'abri de l'eau et à des températures d'au plus 100°C, procédé caractérisé en ce qu'on fait réagir l'acétone, la chloracétone ou la dichloro-1,1 acétone, isolément ou en mélange, à des températures situées dans l'intervalle allant de 10 à 80°C, avec des quantités de chlore au moins égales à la quantité équimolaire, en présence d'iode ou d'un composé iodé soluble dans le mélange réactionnel.

2. Procédé selon la revendication 1, caractérisé en ce qu'on effectue la chloration à des températures situées dans l'intervalle allant de 15 à 50°C.

3. Procédé selon la revendication 1 caractérisé en ce qu'on effectue la chloration avec un léger défaut de chlore.

4. Procédé selon la revendication 3 caractérisé en ce qu'on utilise de 2,6 à 2,9 mol de chlore par mole d'acétone, de 1,6 à 1,9 mol de chlore par mole de monochloracétone mise en jeu, et de 0,3 à 0,8 mol de chlore par mole de dichloro-1,1 acétone mise en jeu.

5. Procédé selon la revendication 1 caractérisé en ce qu'on utilise, comme composé iodé, l'iodacétone la iodo-1 chloro-3 acétone, la dichloro-1,1 iodo-3 acétone, le chlorure d'iode, le trichlorure d'iode ou l'iodure d'hydrogène.

**Claims**

1. Process for the preparation of 1,1,3-trichloroacetone by chlorination of acetone, or the mono- or dichloro-derivatives thereof, with the exclusion of water and at temperatures of up to 100°C, characterized in that, acetone, chloroacetone or 1,1-dichloracetone, on their own or as a mixture, are reacted at temperatures in the range from 10° to 80°C with not more than equimolar amounts of chlorine in the presence of iodine or an iodine-containing compound soluble in the reaction mixture.

2. Process according to Claim 1, characterized in that the chlorination is carried out at temperatures in the range from 15° to 50°C.

3. Process according to Claim 1, characterized in that the chlorination is carried out with a small chlorine deficiency.

4. Process according to Claim 3, characterized in that 2.6 to 2.9 moles of chlorine per mole of acetone, 1.6 to 1.9 moles of chlorine per mole of monochloroacetone employed and 0.3 to 0.8 mole of chlorine per mole of 1,1-dichloroacetone employed are used.

5. Process according to Claim 1, characterized in that iodoacetone, 1-iodo-3-chloroacetone, 1,1-dichloro-3-iodoacetone, iodine chloride, iodine trichloride or hydrogen iodide are used as iodine-containing compounds.